# EUROPÄISCHE PATENTSCHRIFT

(11) **EP 1 060 155 B1**
(45) Veröffentlichungstag und Bekanntmachung des Hinweises auf die Patenterteilung: **21.05.2003**
(21) Anmeldenummer: 99910290.8
(22) Anmeldetag: 26.02.1999
(51) Int. Cl.: C07C 29/80, C07C 45/82, C07C 31/20, C07C 47/19, C07D 307/20, C07C 29/149

(54) **VERFAHREN ZUR DESTILLATION BUTANDIOLHALTIGER GEMISCHE**
METHOD FOR DISTILLING MIXTURES CONTAINING BUTANEDIOL
PROCEDE DE DISTILLATION DE COMPOSITIONS CONTENANT DU BUTANEDIOL

(30) Priorität: 05.03.1998 DE 19809493
(43) Veröffentlichungstag der Anmeldung: 20.12.2000
(73) Patentinhaber: BASF AKTIENGESELLSCHAFT, 67056 Ludwigshafen (DE)
(72) Erfinder: PINKOS, Rolf, D-67098 Bad Dürkheim (DE); FISCHER, Rolf, D-69121 Heidelberg (DE); LIANG, Shelue, D-67071 Ludwigshafen (DE)
(74) Vertreter: Isenbruck, Günter, Dr.
(86) Internationale Anmeldenummer: EP9901250
(87) Internationale Veröffentlichungsnummer: WO99044975

(56) Entgegenhaltungen:
- EP-A- 0 557 786
- WO-A-97/36846
- PATENT ABSTRACTS OF JAPAN vol. 005, no. 180 (C-079), 19. November 1981 & JP 56 104881 A (TORAY IND INC), 20. August 1981

## Beschreibung

Die Erfindung betrifft ein Verfahren zur Destillation eines Gemisches, enthaltend Butan-1,4-diol und 4-Hydroxybutyraldehyd oder dessen cyclische Acetale.

Der Reaktionsaustrag der katalytischen Hydrierung von Maleinsäurederivaten zu Butan-1,4-diol enthält im allgemeinen neben anderen Nebenbestandteilen 4-Hydroxybutyraldehyd, dessen cyclisches Halbacetal 2-Hydroxytetrahydrofuran sowie cyclische Vollacetale des 4-Hydroxybutyraldehyds. Das cyclische Acetal des 4-Hydroxybutyraldehyds mit Butan-1,4-diol, das 2-(4-Hydroxybutoxy)-tetrahydrofüran, läßt sich nur sehr schwer destillativ von Butan-1,4-diol trennen und stellt deshalb eine Quelle für Verunreinigungen für das durch Destillation gewonnene Butan-1,4-diol dar. Es wird daher nach Methoden gesucht, um den Gehalt an 2-(4-Hydroxybutoxy)-tetrahydrofuran in den aufzuarbeitenden butandiolhaltigen Hydrierausträgen zu verringern.

In WO 88/00937 ist ein Verfahren der Hydrierung von Dialkylmaleaten offenbart. Das erhaltene 1,4-Butandiol enthaltende Gemisch wird anschließend durch Destillation aufgetrennt. Die Gegenwart alkalischer Verbindungen während der Destillation wird nicht erwähnt.

In WO 97/36846 ist ein Verfahren beschrieben, bei dem zur Verringerung des Gehalts an 2-(4-Hydroxybutoxy)-tetrahydrofuran in Roh-Butan-1,4-diol dieses in Gegenwart von 0,5 bis 5 Gew.-% Wasser einer katalytischen Hydrierung unterworfen wird. Ein solcher zusätzlicher Hydrierschritt ist aufwendig und verteuert ein Verfahren zur Gewinnung von Butan-1,4-diol erheblich. Außerdem muß zugesetztes Wasser wieder entfernt werden.

In JP-A 09-59191 ist ein Verfahren beschrieben, bei dem 2-Hydroxytetrahydrofuran mit den in Roh-Butan-1,4-diol enthaltenen Alkylalkoholen an einem festen sauren Katalysator zu 2-Alkoxytetrahydrofuran umgesetzt wird, das destillativ abgetrennt wird. Nachteilig an diesem Verfahren ist, daß Butan-1,4-diol an sauren Katalysatoren zum Teil zu Tetrahydrofuran cyclisiert wird, wodurch Ausbeuteverluste an Butan-1,4-diol entstehen. Ferner entsteht in Gegenwart von Butan-1,4-diol auch das Butandiolacetal des 4-Hydroxybutyraldehyds, dessen Bildung gerade vermieden werden soll. Zu bedenken ist dabei ferner, daß der aus dem Dialkylmaleat stammende Alkylalkohol nur in zweifachem molaren Überschuß über Butan-1,4-diol vorliegt, wobei noch hinzukommt, daß letzteres bezüglich der Acetalisierung eine höhere Funktionalität aufweist.

Aufgabe der Erfindung ist es, ein Verfahren zur Destillation der bei der Hydrierung von Maleinsäurederivaten erhaltenen butandiolhaltigen Roh-Gemische bereitzustellen, bei dem ein an 2-(4-Hydroxybutoxy)-tetrahydrofuran besonders armes Butan-1,4-diol als Destillationsprodukt erhalten wird.

Gelöst wird die Aufgabe durch ein Verfahren zur destillativen Trennung eines Gemisches, enthaltend Butan-1,4-diol und mindestens eine weitere Verbindung aus der Gruppe bestehend aus 4-Hydroxybutyraldehyd, dessen cyclischem Halbacetal und dessen cyclischen Vollacetalen mit mindestens einem weiteren Alkohol, wobei in Gegenwart einer alkalisch wirkenden Verbindung destilliert wird.

Prinzipiell ist das erfindungsgemäße Verfahren auf beliebige Gemische anwendbar, sofern das destillativ zu trennende Gemisch zumindest eine der Verbindungen aus der Gruppe bestehend aus 4-Hydroxybutyraldehyd, dessen cyclischem Halbacetal und dessen cyclischen Vollacetalen enthält. Beispielsweise kann von den vorstehend genannten Verbindungen 4-Hydroxybutyraldehyd allein vorliegen. Es können auch der offenkettige Aldehyd, dessen cyclisches Halbacetal und dessen cyclische Vollacatale nebeneinander vorliegen.

Bevorzugt wird das erfindungsgemäße Verfahren zur destillativen Trennung von Gemischen eingesetzt, wie sie bei der Butan-1,4-diol-Synthese durch katalytische Hydrierung von Maleinsäurederivaten anfallen. Mit Maleinsäurederivaten sind Maleinsäureanhydrid, Fumarsäure, Maleinsäuremono- und -diester, Fumarsäuremonound -diester, Bernsteinsäure, Bemsteinsäuremono- und -diester und gamma-Butyrolacton, aber auch Maleinsäure selbst, gemeint. Die genannten Maleinsäurederivate können allein oder als Gemische, in Lösungsmitteln wie Wasser oder Alkoholen, in flüssiger Phase oder in der Gasphase katalytisch hydriert werden. Die Hydrierausträge können neben Butan-1,4-diol u. a. Tetrahydrofüran, gamma-Butyrolacton, Alkohole wie Methanol, Ethanol, Propanol, Butanol, Wasser sowie unumgesetzte Maleinsäurederivate enthalten.

In den erhaltenen Gemischen liegen im allgemeinen 4-Hydroxybutyraldehyd, dessen cyclisches Halbacetal 2-Hydroxytetrahydrofuran, sowie dessen cyclische Vollacetale nebeneinander vor.

In einer bevorzugten Variante des erfindungsgemäßen Verfahrens enthält das zu trennende Gemisch cyclische Vollacetale des 4-Hydroxybutyraldehyds mit weiteren Alkoholen. Bevorzugte weitere Alkohole sind die bei der katalytischen Hydrierung von Maleinsäurederivaten, beispielsweise den Dialkylmaleaten, als Nebenprodukte gebildete Alkohole, wie Methanol, Ethanol, n-Propanol, n-Butanol. Besonders bevorzugte weitere Alkohole sind die Esteralkohole, die in den üblicherweise als Edukte für die katalytische Hydrierung eingesetzten Dialkylmaleaten gebunden vorliegen, beispielsweise Methanol, Ethanol, Propanol und n-Butanol.

Bevorzugt enthält das zu trennende Gemisch cyclische Vollacetale des 4-Hydroxybutyraldehyds mit weiteren Alkoholen, die einen niedrigeren Siedepunkt als Butan-1,4-diol aurweisen. Beispiele sind Methanol, Ethanol, Propanol und n-Butanol.

In einer besonders bevorzugten Variante des erfindungsgemäßen Verfahrens enthält das zu trennende Gemisch den im cyclischen Vollacetal des 4-Hydroxybutyraldehyds gebundenen weiteren Alkohol auch in freier Form. Beispiele hierfür sind Gemische, in denen 2-Methoxytetrahydrofuran oder 2-Ethoxytetrahydrofuran neben Methanol bzw. Ethanol vorliegen. Solche Gemische fallen beispielsweise bei der Hydrierung von Maleinsäuredimethylester bzw. - diethylester an.

Die zu trennenden Gemische können ferner 2-(4-Hydroxybutoxy)-tetrahydrofuran enthalten. Vorzugsweise enthalten die zu trennenden Gemische die letztgenannte Verbindung.

In einer insbesondere bevorzugten Variante des erfindungsgemäßen Verfahrens enthalten die zu trennenden Gemische neben 2-(4-Hydroxybutoxy)-tetrahydrofuran das 2-Methoxytetrahydrofuran oder 2-Ethoxytetrahydrofuran sowie Methanol bzw. Ethanol, vor allem, wenn Maleinsäuredimethyl- bzw. --diethylester hydriert wurden.

Die Destillation des zu trennenden Gemisches wird in Gegenwart alkalisch wirkender Verbindungen durchgeführt. Alkalisch wirkend heißt, daß der Protonierung der in dem betreffenden Gemisch enthaltenen Alkohole, Aldehyde, Halb- und Vollacetale entgegengewirkt und damit die Konzentration der entsprechenden protonierten Spezies verringert wird. Es können in dem zu trennenden Gemisch lösliche oder unlösliche alkalisch wirkende Verbindungen eingesetzt werden. Bevorzugt werden in dem zu trennenden Gemisch lösliche alkalisch wirkende Verbindungen eingesetzt. Bevorzugte alkalisch wirkende Verbindungen sind Ammoniak, Amine, Alkali- oder Erdalkaliverbindungen, bevorzugt die Oxide, Hydroxide, Carbonate, Alkoholate, wie die Methanolate, Ethanolate, Propanolate und Butanolate, und Carboxylate, wie die Formiate, Acetate und Propionate, jeweils des Lithiums, Natriums, Kaliums, Magnesiums und Calciums.

Im allgemeinen liegt die Konzentration der alkalisch wirkenden Verbindung in dem zu destillierenden Gemisch im zeitlichen Mittel zwischen 0,0001 und 5, bevorzugt zwischen 0,001 und 1, besonders bevorzugt zwischen 0,01 und 0,5 Gew.-%.

Die alkalisch wirkende Komponente kann unverdünnt oder in einem Lösungsmittel, beispielsweise in Wasser oder Alkohol, gelöst zugegeben werden. Vorzugsweise wird die alkalisch wirkende Komponente als wäßrige, wäßrig-alkoholische oder alkoholische Lösung zugegeben. Wird das erfindungsgemäße Verfahren zur Aufarbeitung von Hydrierausträgen bei der Butan-1,4-diol-Synthese eingesetzt, so erfolgt die Zugabe im allgemeinen nach dem Hydrierschritt.

Die Destillation kann in einer oder mehreren Stufen, beispielsweise in einer oder mehreren Destillationskolonnen, durchgeführt werden, wobei kontinuierlich oder diskontinuierlich gearbeitet werden kann. Bevorzugt wird die Destillation in zwei oder mehreren Stufen kontinuierlich durchgeführt. Enthält das zu destillierende Gemisch neben Butan-1,4-diol einen weiteren Alkohol mit einem niedrigeren Siedepunkt, wie es im allgemeinen in den Hydrierausträgen der Butan-1,4-diol-Synthese der Fall ist, so wird die Destillation in vorteilhafter Weise in mindestens zwei Stufen durchgeführt, wobei in einer ersten Stufe der weitere Alkohol und in einer zweiten Stufe Butan-1,4-diol abdestilliert werden. Es kann auch in mehr als zwei Stufen destilliert werden. Beispielsweise können in einer ersten Stufe Leichtsieder, wie Methanol oder Ethanol oder Tetrahydrofuran, in einer zweiten Stufe Nachsieder, wie Butyrolacton und Bernsteinsäurediester und in einer dritten Stufe Butan-1,4-diol von Hochsiedern wie Butandiolbernsteinsäureester abgetrennt werden. Wird in mehreren Stufen destilliert, kann die alkalisch wirkende Verbindung - zumindest teilweise - auch erst nach erfolgter Leichtsieder-Abtrennung zugegeben werden. Der bei der letzten Destillationsstufe im Sumpf verbleibende Hochsieder-Anteil, der auch die alkalisch wirkende Verbindung enthält, kann in die erste Destillationsstufe zurückgeführt werden.

Mit dem erfindungsgemäßen Verfahren wird sehr reines Butan-1,4-diol erhalten. Bei der Destillation der Butandiol-haltigen Reaktionsausträge der Hydrierung von Dialkylmaleaten werden Gehalte an 2-(4-Hydroxybutoxy)-tetrahydrofuran von unter 2000 ppm, bevorzugt von unter 1000 ppm, besonders bevorzugt von unter 500 ppm erzielt.

Die Erfindung wird durch die nachstehenden Beispiele näher erläutert.

Als destillativ zu trennendes Gemisch wird der Reaktionsaustrag der Gasphasenhydrierung von Maleinsäuredimethylester an einem Cu/Mn/Al-Katalysator (T 4489 der Süd-Chemie AG, München, DE) bei 60 bar und 190°C eingesetzt. Der Hydrieraustrag enthält ca. 51 Gew.-% Butan-1,4-diol, 41 Gew.-% Methanol, ca. 0,05 Gew.-% 2-Methoxyhytetradrofuran, ca. 0,05 Gew.-% 2-Hydroxytetrahydrofuran, ca. 0,05 Gew.-% 2-(4-Hydroxybutoxy)-tetrahydrofuran sowie 1 Gew.-% Tetrahydrofuran, 3 Gew.-% gamma-Butyrolacton, 1 Gew.-% Butanol und 1 Gew.-% Wasser. Der Reaktionsaustrag wurde für das erfindungsgemäße Beispiel und das Vergleichsbeispiel getrennt.

### Beispiel

0,8 g KOH werden in 745 g des Reaktionsaustrages gelöst. Anschließend werden bei Atmosphärendruck vorwiegend Methanol und Tetrahydrofuran abdestilliert. Danach wird über eine Füllkörperkolonne mit Rücklaufteiler (Rücklaufverhältnis = 10) bei 40 mbar fraktioniert destilliert. Bei einer Sumpftemperatur von 146°C und einer Kopftemperatur von 136 °C wird am Kopf der Kolonne Butan-1,4-diol mit einem Gehalt an 2-(4-Hydroxybutoxy)-tetrahydrofüran von 300 bis 400 ppm erhalten.

### Vergleichsbeispiel

Es wird wie im Erfindungsbeispiel gearbeitet, jedoch ohne Zusatz von KOH. Der Gehalt des erhaltenen Butan-1,4-diols an 2-(4-Hydroxybutoxy)-tetrahydrofuran beträgt 900 bis 1000 ppm.

## Patentansprüche

1. Verfahren zur destillativen Trennung eines Gemisches, enthaltend Butan-1,4-diol und mindestens eine weitere Verbindung aus der Gruppe bestehend aus 4-Hydroxybutyraldehyd, dessen cyclischem Halbacetal und dessen cyclischen Vollacetalen mit mindestens einem weiteren Alkohol, wobei in Gegenwart einer alkalisch wirkenden Verbindung destilliert wird.

2. Verfahren nach Anspruch 1, **dadurch gekennzeichnet, daß** das zu trennende Gemisch ein cyclisches Vollacetal des 4-Hydroxybutyraldehyds mit einem weiteren Alkohol enthält, wobei dieser Alkohol eine niedrigere Siedetemperatur als Butandiol aufweist.

3. Verfahren nach Anspruch 2, **dadurch gekennzeichnet, daß** das zu trennende Gemisch den weiteren Alkohol auch in freier Form enthält.

4. Verfahren nach Anspruch 3, **dadurch gekennzeichnet, daß** in mindestens zwei Destillationsstufen in einer ersten Stufe der weitere Alkohol abdestilliert und in einer zweiten Stufe Butandiol abdestilliert wird.

5. Verfahren nach einem der Ansprüche 1 bis 4, **dadurch gekennzeichnet, daß** die alkalisch wirkende Verbindung im zu trennenden Gemisch löslich ist.

6. Verfahren nach einem der Ansprüche 1 bis 5, **dadurch gekennzeichnet, daß** die alkalisch wirkende Verbindung ausgewählt ist aus der Gruppe bestehend aus Oxiden, Hydroxiden, Carbonaten, Carboxylaten und Alkoholaten des Lithiums, Natriums, Kaliums, Magnesiums und Calciums.

7. Verfahren nach einem der Ansprüche 1 bis 6, **dadurch gekennzeichnet, daß** ein bei der Hydrierung von Maleinsäurederivaten anfallendes Gemisch destillativ getrennt wird.

## Claims

1. A process for the separation by distillation of a mixture comprising 1,4-butanediol and at least one further compound from the group consisting of 4-hydroxybutyraldehyde, its cyclic hemiacetal and its cyclic full acetals with at least one further alcohol, which comprises carrying out the distillation in the presence of an alkaline compound.

2. A process as claimed in claim 1, wherein the mixture to be separated comprises a cyclic full acetal of 4-hydroxybutyraldehyde with a further alcohol which has a lower boiling point than butanediol.

3. A process as claimed in claim 2, wherein the further alcohol is additionally present in free form in the mixture to be separated.

4. A process as claimed in claim 3, wherein, in at least two distillation stages, the further alcohol is distilled off in a first stage and butanediol is distilled off in a second stage.

5. A process as claimed in any of claims 1 to 4, wherein the alkaline compound is soluble in the mixture to be separated.

6. A process as claimed in any of claims 1 to 5, wherein the alkaline compound is selected from the group consisting of oxides, hydroxides, carbonates, carboxylates and alkoxides of lithium, sodium, potassium, magnesium and calcium.

7. A process as claimed in any of claims 1 to 6, wherein a mixture formed in the hydrogenation of maleic acid derivatives is separated by distillation.

## Revendications

1. Procédé de séparation par distillation d'un mélange contenant du butane-1,4-diol et au moins un autre composé de l'ensemble comprenant le 4-hydroxybutyraldéhyde, son semi-acétal cyclique et ses acétals cycliques complets, avec au moins un autre alcool, la distillation étant effectuée en présence d'un composé à effet alcalin.

2. Procédé selon la revendication 1, **caractérisé en ce que** le mélange devant être séparé contient un acétal cyclique complet du 4-hydroxybutyraldéhyde avec un autre alcool, cet alcool ayant une température d'ébullition plus basse que le butanediol.

3. Procédé selon la revendication 2, **caractérisé en ce que** le mélange devant être séparé contient aussi l'autre alcool sous forme libre.

4. Procédé selon la revendication 3, **caractérisé en ce que**, dans au moins deux étapes de distillation, on chasse l'autre alcool par distillation dans une première étape, et on chasse le butanediol par distillation dans une deuxième étape.

5. Procédé selon l'une des revendications 1 à 4, **caractérisé en ce que** le composé à effet alcalin est soluble dans le mélange devant être séparé.

6. Procédé selon l'une des revendications 1 à 5, **caractérisé en ce que** le composé à effet alcalin est choisi dans l'ensemble comprenant les oxydes, les hydroxydes, les carbonates, les carboxylates et les alcoolates de lithium, de sodium, de potassium, de magnésium et de calcium.

7. Procédé selon l'une des revendications 1 à 6, **caractérisé en ce qu'**on sépare par distillation un mélange obtenu lors de l'hydrogénation de dérivés de l'acide maléique.
